# FASCICULE DE BREVET EUROPEEN

(11) **EP 0 779 284 B1**
(45) Date de publication et mention de la délivrance du brevet: **10.07.2002**
(21) Numéro de dépôt: 96402672.8
(22) Date de dépôt: 09.12.1996
(51) Int. Cl.: C07D 295/12, C07D 211/14, C07D 211/70, A61K 31/445

(54) **Nouveaux dérivés de 2-naphthamides et leurs applications thérapeutiques comme agonistes des récepteurs D3**
Neue Naphthamid-Derivate und ihre therapeutische Verwendung als D3-Rezeptor Agonisten
Novel 2-naphthamide derivatives and their use in therapy as D3 receptor agonists

(30) Priorité: 11.12.1995 FR 9514654
(43) Date de publication de la demande: 18.06.1997
(73) Titulaire: INSTITUT NATIONAL DE LA SANTE ET DE LA RECHERCHE MEDICALE (INSERM), 75013 Paris (FR); SOCIETE CIVILE BIOPROJET, F-75003 Paris (FR)
(72) Inventeur: Wermuth, Camille-Georges, 67100 Strasbourg (FR); Mann, André, 67540 Ostwald (FR); Garrido, Fabrice, 67100 Strasbourg (FR); Lecomte, Jeanne-Marie, 75003 Paris (FR); Schwartz, Jean-Charles, 75014 Paris (FR); Sokoloff, Pierre, 95130 Le Plessis Bouchard (FR)
(74) Mandataire: Bernasconi, Jean

(56) Documents cités:
- EP-A- 0 539 281
- WO-A-93/21179
- WO-A-93/23035
- US-A- 5 254 552
- US-A- 5 395 835

## Description

La présente invention concerne de nouveaux composés chimiques dérivés de 2-naphtamides ainsi que leurs applications thérapeutiques notamment comme agents dopaminergiques sélectifs.

De nombreux dérivés de phénylpipérazines sont connus et utilisés pour leur activité sur le système nerveux central, en particulier pour leurs propriétés neuroleptiques.

Les phénylpipérazines sont connues essentiellement comme agents sérotoninergiques.

En ce qui concerne les récepteurs de la dopamine, il a été montré que certains dérivés arylpipérazines présentent une affinité plus élevée pour le récepteur D₃ de la dopamine, par rapport aux autres récepteurs de celle-ci, (MURRAY P.J. et al., Bioorganic & Medicinal Chemistry Letters, vol. 5 n° 3, pp 219-222 (1995)).

Selon ce document, ces composés, qui présentent une certaine sélectivité vis-à-vis du récepteur D₃ de la dopamine par rapport aux autres récepteurs, pourraient être utilisés pour vérifier l'hypothèse qu'un antagoniste sélectif du récepteur D₃ de la dopamine pourrait fournir un agent antipsychotique efficace exempts d'effets secondaires extrapyramidaux.

Par ailleurs, il a été montré que certains dérivés de naphtamides se comportaient comme de purs antagonistes du récepteur D₃ et pouvaient à ce titre être utilisés pour la préparation de médicaments antagonistes de la dopamine par blocage du récepteur D₃ (Demande de brevet français N°91 13103).

Récemment, des dérivés naphtamides d'arylpiperazines ont été décrits dans le brevet US-A-5 395 835 également comme antagonistes sélectifs du récepteur D₃ de la dopamine. Ces composés sont utiles comme agents antipsychotiques et pour le traitement des troubles liés au blocage dopaminergique.

C'est dans cet état des connaissances que les inventeurs ont mis en évidence, de façon tout-à-fait surprenante et inattendue, que des dérivés 2-naphtamides de formule (I) donnée ci-dessous, présentaient une forte affinité pour les récepteurs dopaminergiques et notamment pour le récepteur D₃ et que de manière seléctive, ils se comportaient comme de puissants agonistes partiels de la dopamine au récepteur D₃.

Ainsi, la présente invention a pour objet des dérivés de 2-naphtamides, sous forme de bases ou de sels, répondant à la formule générale (I) : 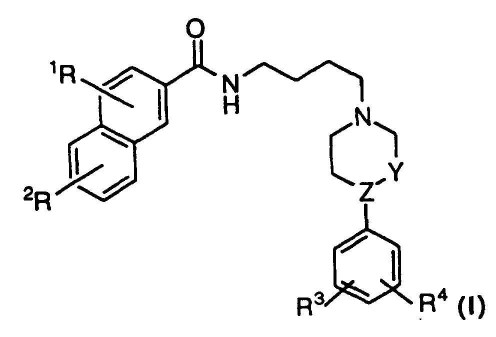 dans laquelle :
- l'entité Z-Y représente un groupement N-CH₂, C=CH ou CH-CH₂ ;
- R¹ représente un atome d'hydrogène, de fluor, de brome ou d'iode, ou un groupement hydroxy, méthoxy, nitrile ou nitro ;
- R² représente un atome d'hydrogène ou de brome, ou un groupement hydroxy, méthoxy, nitrile ou nitro ;
les substituants R₁ et R₂ étant situés tous les deux sur le même noyau du motif naphtamide ou chacun sur un des noyaux ;
- R³ et R⁴ peuvent être identiques ou différents et représentent chacun indépendemment un atome d'hydrogène ou de chlore, ou un groupement méthoxy ou méthyle.

L'invention a encore pour objet des compositions pharmaceutiques comprenant une quantité thérapeutiquement efficace d'au moins un dérivé de formule (I) précitée, sous forme de base ou de sel pharmaceutiquement acceptable, en combinaison avec un véhicule ou excipient pharmaceutiquement acceptable.

Elle concerne encore des médicaments agissant comme agonistes partiels du récepteur D₃ de la dopamine comprenant à titre de principe actif, au moins un dérivé de formule (I) précitée ainsi que l'utilisation desdits dérivés pour la préparation de tels médicaments.

Les dérivés selon l'invention sont représentés par la formule générale (I). Ces composés sont nouveaux.

Dans la littérature, on peut trouver des dérivés naphtamides d'arylpipérazines (US-A-5 395 835 précité) mais dans ces composés, le motif pipérazine est séparé du motif naphtamide par une chaîne à 2 atomes de carbone alors que, selon l'invention, la chaîne présente 4 atomes de carbone.

Les dérivés selon l'invention peuvent se présenter sous forme de bases libres ou sous forme de sels, en particulier sous forme de sels d'addition avec des acides physiologiquement acceptables et l'invention s'étend également à ces différentes formes.

Selon l'invention, les dérivés pour lesquels l'entité Z-Y représente un groupement N-CH₂ constituent des dérivés préférés.

A titre de dérivés particulièrement préférés selon l'invention, on peut citer les composés suivants :
- N-[4-(4-(2-méthoxyphényl) pipérazinyl) butyl] 1-méthoxy 4-nitro 2-naphtamide ;
- N-[4-(4-phényl 1,2,3,6-tétrahydropyridinyl) butyl] 2-naphtamide ;
- N-[4-(4-phényl pipéridinyl) butyl] 2-naphtamide ;
- N-[4-(4-(2-méthoxyphényl) pipérazinyl) butyl] 1-méthoxy 4-cyano 2-naphtamide ;
- N-[4-(4-(2-méthoxyphényl) pipérazinyl) butyl] 2-naphtamide ;
- N-[4-(4-(2-chlorophényl) pipérazinyl) butyl] 3-méthoxy 2-naphtamide ;
- N-[4-(4-(2-chlorophényl) pipérazinyl) butyl] 2-naphtamide ;
- N-[4-(4-(3-chlorophényl) pipérazinyl) butyl] 2-naphtamide ;
- N-[4-(4-phényl pipérazinyl) butyl] 2-naphtamide ;
- Oxalate du N-[4-(4-(2-méthoxyphényl) pipérazinyl) butyl] 1-méthoxy 2-naphtamide ;

Les dérivés de formule (I) selon la présente invention peuvent être préparés par des méthodes connues (W.Adcock et al., Aust. *J. Chem.,* **1965,** 18, 1351).

La partie acide (acide 2-naphtoïque) convenablement substituée, est transformée en anhydride mixte par le chloroformiate d'isobutyle dans l'acétone ou tout autre solvant, en milieu basique et mis en réaction avec l'amine souhaitée comme indiqué dans le schéma réactionnel suivant :

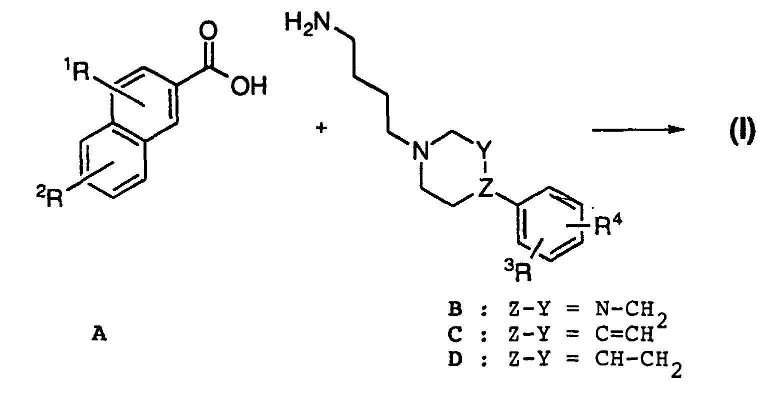

D'autres méthodes d'activation de la fonction carboxylique peuvent également être utilisées ; en fait toutes les méthodes de préparation d'amide conviennent, y compris l'emploi des chlorures d'acide correspondant.

Les amino-pipérazines de type B sont obtenues par des méthodes conventionnelles souvent au départ de phénylpiperazines commercialement disponibles, par alkylation au moyen de chlorobutyronitrile en milieu basique dans un solvant alcoolique. Puis la fonction nitrile est réduite en amine primaire soit par LiAlH₄ soit par hydrogénation catalytique en présence de palladium (Pd) sur charbon.

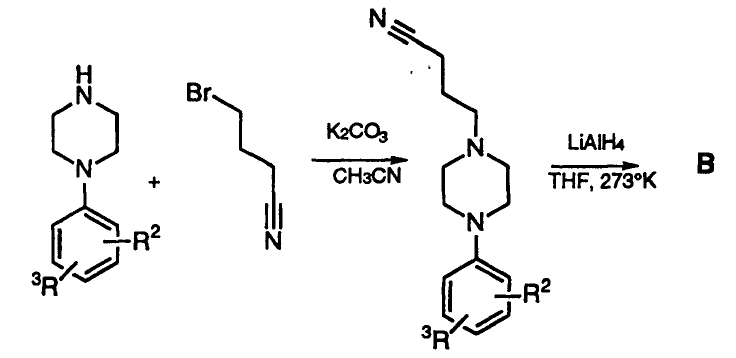

Les aminobutylphényltetrahydropyridines de type C ou les aminobutylphénylpipéridines de type D sont également obtenues par des méthodes conventionnelles à partir soit de produits commerciaux, soit de N-Boc 4-pipéridone (où Boc signifie tert-butoxycarbonyle) que l'on fait réagir avec un phényl magnésien puis que l'on deshydrate pour obtenir la tetrahydropyridine correspondante. Par hydrogénation catalytique de cette dernière, on obtient la pipéridine correspondante. La protection t-butoxycarbonyle (Boc) est hydrolysée en milieu acide et l'azote est alkylé par du bromobutyronitrile en milieu basique de la même manière que précédemment. Le schéma de préparation des composés C et D est donné ci-dessous :

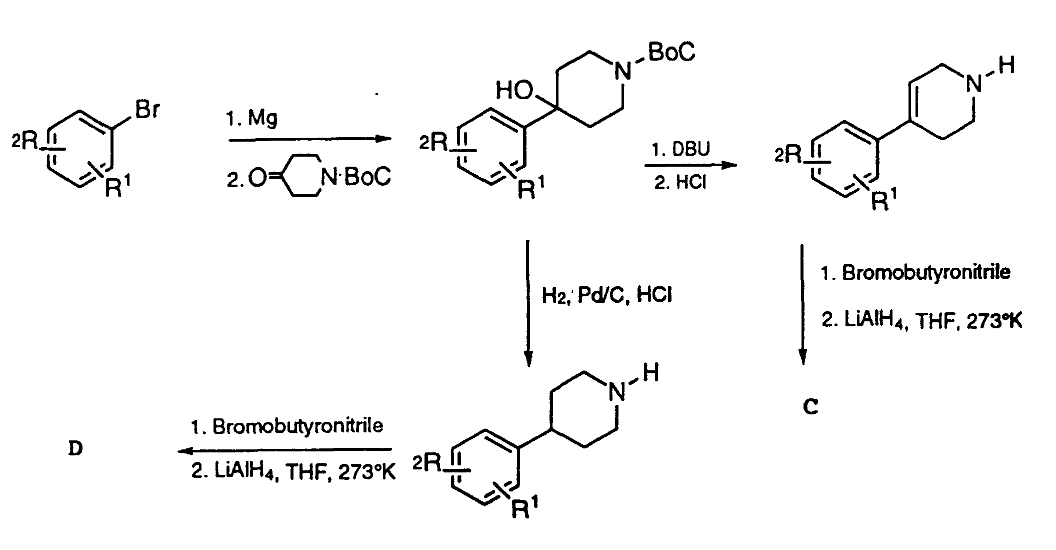

L'invention va être décrite plus en détail ci-après à l'aide des exemples suivants donnés à titre illustratif et non limitatif.

### EXEMPLES

### Exemple 1 : Préparation du N-[4-(4-(2-méthoxyphényl) pipérazinyl) butyl] 1-méthoxy 4-nitro 2-naphtamide (Do 885)

Dans un bicol de 25 ml, dissoudre 120 mg d'acide 1-méthoxy 4-nitro naphtalène 2-carboxylique A (préparé comme indiqué ci-après au a)) dans 15 ml d'acétone anhydre. Rajouter 2,5 équivalents de triéthylamine, refroidir le milieu à -15°C au moyen d'un bain carboglace-acétone. Rajouter ensuite au mélange, 1,05 équivalent de chloroformiate d'isobutyle et laisser réagir 1 heure à -15°C. Rajouter ensuite 1,05 équivalent de N-4-aminobutyl N'-(2-méthoxyphényl) pipérazine B (préparé comme indiqué ci-après au b)) et laisser réagir 2 heures à température ambiante sous atmosphère inerte. Filtrer le chlorhydrate de triéthylamine et évaporer le filtrat à sec. Reprendre le résidu dans un peu d'acétate d'éthyle et purifier le naphtamide par chromatographie sur colonne de silice (éluant : AcOEt-MeOH 90/10). Cristalliser le naphtamide dans du diéthyléther. On obtient 85 mg de cristaux jaunes. R = 37 %.
TF=109°C. RMN ¹H (CDCl₃) : 1,59-1,73 (m, 2H, -CH₂-), 2,47-2,66 (m, 6H, -H₂C-CH₂-CH₂-), 3,02-3,20 (m, 4H, -CH₂-CH₂-), 3,58-3,61 (m, 4H, -H₂C-CH₂-), 3,86 (s, 3H, OCH₃, phényl), 4,08 (s, 3H, OCH₃, naphtyl), 6,79-7,03 (m, 4H, -HC-CH-CH-CH-, phényl), 7,68-7,85 (m, 2H, -HC-CH-), 8,27-8,31 (d, 1H, -CH-), 8,62-8,66 (d, 1H, -CH-), 8,87 (s, 1H, -CH-). Analyse C₂₇H₃₂N₄O₅
Cal. C % 65,84 ; H % 6,55 ; N % 11,37
Tr. C % 66,25 ; H % 6,43 ; N % 11,12

### a) Préparation de l'acide 1-méthoxy 4-nitro 2-naphtoïque (A)

### - Ester méthylique de l'acide 1-méthoxy 2-naphtoïque

Mettre en suspension 1,88 g (10 mmoles) d'acide 1-hydroxy 2-naphtoïque dans 50 ml de méthyléthylcétone. Additionner 2,76 g (2 équivalents) de carbonate de potassium anhydre, puis, 2,51 g (2 équivalents) de diméthylsulfate en solution dans le solvant au goutte-à-goutte. Chauffer le mélange à reflux sous agitation pendant 1 nuit, puis, après refroidissement, filtre l'excès de carbonate de potassium et concentrer le filtrat. Reprendre le résidu d'évaporation dans l'eau et extraire plusieurs fois avec de l'acétate d'éthyle. Laver la phase organique avec de l'eau, sécher sur du sulfate de sodium puis évaporer. Purifier par chromatographie sur colonne de silice (éluant : Hexane-Acétate d'éthyle 90/10). On obtient une huile dorée. R = 82 %.
RMN-¹H (CDCl₃) : 4,00 (3H, s, CO₂CH₃), 4,08 (3H, s, OCH₃), 7,55-7,63 (3H, m, HC-CH-CH), 7,83-7,90 (2H, m, HC-CH), 8,27-8,32 (1H, m, CH). RMN ¹³C (CDCl₃) : 52,1 (OCH₃), 58,3 (OCH₃), 166,4 (C=O), 158,1 (C-O,α), 119,0 (C-C=O,β), 126,3 (C-H,β), 123,4 (C-H,α), 128,2 (C-H,α), 128,4 (C-H,β), 127,7 (C-H,β) 127,6 (C-H,α), 128,6 (C-C,γ), 136,6 (C-C,γ)

### - Ester méthylique de l'acide 1-méthoxy 4-nitro 2-naphtoïque

Dissoudre 2 g d'ester méthylique de l'acide 1-méthoxy 2-naphtoïque dans 15 ml d'acide acétique cristallisable. Rajouter au goutte-à-goutte 1,2 équivalent d'acide nitrique concentré en solution dans de l'acide acétique cristallisable et laisser réagir pendant 4 heures à température ambiante sous bonne agitation. Hydrolyser lentement le milieu réactionnel dans de la glace. L'ester méthylique de l'acide 1-méthoxy 4-nitro 2-naphtoïque précipite. Filtrer le précipité, le laver plusieurs fois avec de l'eau glacée puis, redissoudre dans de l'acétate d'éthyle, laver avec de l'eau, puis, avec une solution saturée de carbonate de potassium *(note 1)* pour éliminer l'acide acétique résiduel et enfin, laver avec de l'eau. Sécher sur du sulfate de sodium et concentrer le milieu. Purifier par chromatographie sur colonne de silice (éluant : Hexane-CH₂Cl₂ 60/40). Cristaux jaunes. R = 89 %. T. Fusion : 110°C. RMN- ¹H (CDCl₃) : 4,02 (3H, s, -CO₂CH₃), 4,14 (3H, s, -OCH₃), 7,65-7,86 (2H, m, -HC-CH), 8,39-8,43 (1H, dd, -CH), 8,62-8,66 (1H, dd, -CH), 8,74 (1H, s, -CH). RMN ¹³C (CDCl₃) : 52,7 (-OCH₃), 64,0 (-OCH₃), 164,5 (>C=O), 162,9 (>C-O, α), 116,9 (>C-C=O,β), 124,4 (>C-H,β), 129,5 (>C-NO₂,α), 141,5 (>C-C,γ), 123,6 (>C-H,α), 131,8 (>C-H, β), 127,9 (>C-H,β), 126,6 (>C-H,α), 128,2 (>C-C,γ).
*Note 1 : Il est préférable de ne pas utiliser d'hydroxyde de sodium car il se forme un complexe très coloré difficile à éliminer. Il est important de bien laver le précipité au préalable pour éliminer le plus d'acide possible car la neutralisation de l'acide acétique par K*_{*2*}*CO*_{*3*} *dégage du dioxyde de carbone.*

### - Acide 1-méthoxy 4-nitro 2-naphtoïque (A)

Dissoudre 750 mg d'ester méthylique de l'acide 1-méthoxy 4-nitro 2-naphtoïque dans 20 ml de méthanol. Rajouter 1,5 équivalent d'hydrogénocarbonate de sodium et chauffer le mélange à reflux pendant 10 heures. Concentrer le milieu et reprendre le résidu avec de l'eau. Extraire avec de l'éther pour éliminer les impuretés organiques et acidifier la phase aqueuse jusqu'à pH = 2 avec de l'acide chlorhydrique 5N. On obtient un précipité blanc-jaunâtre. Extraire avec de l'acétate d'éthyle, laver 3 fois avec de l'eau, sécher sur du sulfate de sodium et concentrer le milieu. Cristalliser l'acide dans du pentane. Purifier par recristallisation à chaud dans de l'eau et addition de charbon activé pour éliminer les impuretés. cristaux-blanc-jaunâtres. R = 94 %.

### b) Préparation de la N-4-aminobutyl N'-(2-méthoxyphényl) pipérazine (B)

### - N-(2-méthoxyphényl) N'-(3-cyanopropyl) pipérazine

Mettre en suspension 8 g de chlorhydrate de N'-(2-méthoxyphényl) pipérazine dans 150 ml d'acétonitrile. Rajouter 2,5 équivalents de carbonate de potassium anhydre, puis, 1,05 équivalent de 4-bromobutyronitrile au goutte-à-goutte en solution dans de l'acétonitrile. Chauffer le mélange à reflux pendant 10 heures, puis, filtrer et concentrer le filtrat. Reprendre le résidu dans de l'acétate d'éthyle et laver 3 fois avec de l'eau. Extraire la phase organique avec une solution d'acide chlorhydrique 1M et laver la phase acidulée avec de l'acétate d'éthyle. Neutraliser la phase acidulée avec de l'ammoniaque à 28 % jusqu'à pH>11. Extraire avec de l'acétate d'éthyle, laver la phase organique avec de l'eau, la sécher sur du sulfate de sodium anhydre et concentrer le milieu. Cristalliser le nitrile dans de l'hexane et recristalliser à chaud dans ce même solvant. On obtient 6,8 g de nitrile. R = 75 %.
TF=74°C. RMN ¹H (CDCl₃) : 1,80-1,94 (m, 2H, -CH₂-), 2,43-2,57 (m, 4H, -CH₂-CH₂-), 2,62-2,67 (t large, 4H, -CH₂-N<), 3,09 (s large, 4H, >N-CH₂-), 3,87 (s, 3H, -OCH₃), 6,85-7,04 (m, 4H, -CH-CH-CH-CH-). RMN ¹³C (CDCl₃) : 14,9 (-CH₂-CH₂-C,N), 22,7 (-CH₂-CN), 50,5 (-CH₂-N-(CH₂)₂-), 53,2 (>C-N-(CH₂)₂-), 55,3 (-OCH₃), 56,3 (>N-CH₂-), 111,1 (-CH-C-OCH₃), 118,1 (-CH-CH-C-OCH₃), 119,8 (-CN), 120,9 (-CH-C-N<), 122,9 (-CH-CH-C-N<), 141,1 (>C-N<), 152,2 (-C-OCH₃).

### - N-4-aminobutyl N'-(2-méthoxyphényl) pipérazine (B)

Mettre en suspension 1,2 g d'hydrure double de lithium et d'aluminium dans 50 ml de diéthyléther anhydre (fraichement distillé sur sodium) par petites portions (dissolution exothermique). Rajouter 5 g de N'-(2-méthoxyphényl) N-(3-cyanopropyl) pipérazine en solution dans du tétrahydrofuranne (THF) anhydre au goutte-à-goutte, puis, chauffer sous -reflux pendant 2 heures. Hydrolyser le milieu avec un mélange de 5 ml d'eau dans 25 ml de THF et laisser reposer une nuit pour permettre au précipité de s'agglomérer. Filtrer le précipité sur célite, sécher le filtrat sur du sulfate de sodium anhydre et concentrer le filtrat.
R = 81 %. RMN ¹³C (CDCl₃) : 24,3 (-CH₂-CH₂-CH₂- NH₂), 31,9 (-CH₂-CH₂-NH₂), 42,2 (-CH₂-NH₂), 50,6 (-CH₂-N-(CH₂)₂-), 53,4 (>C-N-(CH₂)₂-), 55,3 (-OCH₃), 58,6 (>N-CH₂-), 111,1 (-CH-C-OCH₃), 118,1 (-CH-CH-COCH₃), 120,9 (-CH-C-N<), 122,8 (-CH-CH-C-N<), 141,3 (>C-N<), 152,2 (-C-OCH₃).

### Exemple 2 : Préparation du N-[4-(4-phényl 1,2,3,6-tetrahydropyridinyl) butyl] 2-naphtamide (Do 911)

Mettre en suspension 250 mg d'acide naphtalène 2-carboxylique A (acide 2-naphtoïque) dans 20 ml de dichlorométhane anhydre (fraîchement distillé sur CaH₂). Rajouter 1,2 équivalent de chlorure d'oxalyle à O°C puis, 2 gouttes de diméthylformamide anhydre (pour catalyser la réaction de chloruration) puis, laisser réagir à température ambiante pendant 1 heure sous atmosphère inerte (argon) et forte agitation. Concentrer le milieu pour éliminer le solvant et l'excès de chlorure d'oxalyle puis, redissoudre le chlorure d'acide formé dans 20 ml de dichlorométhane. Rajouter 1,05 équivalents de N-4-aminobutyl 4-phényl 1,2,3,6-tetrahydropyridine C (préparé comme indiqué ci-après au c)) et laisser réagir 2 heures à température ambiante sous atmosphère inerte (argon). Concentrer le milieu et reprendre le résidu dans une solution aqueuse d'acide chlorhydrique 3M. Laver la phase acidulée avec de l'acétate d'éthyle puis, neutraliser la phase aqueuse avec une solution d'ammoniaque à 32 % jusqu'à pH>11. Extraire avec de l'acétate d'éthyle, laver plusieurs fois la phase organique avec de l'eau, sécher sur du sulfate de sodium et concentrer le milieu. Cristalliser le résidu dans un mélange éther hexane 50/50.

On obtient 280 mg de cristaux blancs. R = 50 %.
TF = 172 °C. RMN ¹H (CDCl₃): 1,73-1,83 (m, 4H, -H₂C-CH₂-), 2,51-2,58 (t, 4H, -H₂C-CH₂-), 2,69-2,75 (t, 2H, -CH₂-), 3,11-3,16 (q, 2H, -CH₂-), 3,52-3,58 (2H, q, -CH₂-), 6,01-6,04 (1H, t, -CH=), 7,24-7,35 (5H, m, -(CH)₅<, phényl), 7,39-7,55 (m, 2H, >HC-CH<, naphtyl), 7,78-7,85 (4H, m, >HC-CH-CH-CH<, naphtyl), 8,24 (1H, s, >CH-, naphtyl).

### c) Préparation de la N-4-aminobutyl 4-phényl 1,2,3,6-tetrahydropyridine (C)

### - 4-(4-phényl 1,2,3,6-tetrahydropyridinyl) butyronitrile

Dans un ballon de 250 ml, introduire 5 g de chlorhydrate de 4-phényl 1,2,3,6-tetrahydropyridine (composé très toxique : New Engl. J. Med., 1983, 309, 310 ; Science, 1983, 219, 979 ; Psychiatry Res. 1979, 1, 249) puis 100 ml d'acétonitrile. Rajouter 2,5 équivalents de carbonate de potassium anhydre, puis, 1,05 équivalent de 4-bromobutyronitrile au goutte-à-goutte en solution dans de l'acétonitrile. Chauffer le mélange à reflux pendant 16 heures, puis, filtrer, laver le précipité avec de l'acétone et concentrer le filtrat. Reprendre le résidu dans de l'acétate d'éthyle et laver 3 fois avec de l'eau. Extraire la phase organique avec une solution d'acide chlorhydrique 3M et laver la phase acidulée avec de l'acétate d'éthyle. Neutraliser la phase acidulée avec de l'ammoniaque à 28 % jusqu'à pH>11. Extraire avec de l'acétate d'éthyle, laver la phase organique avec de l'eau, la sécher sur du sulfate de sodium anhydre et concentrer le milieu. Purifier le nitrile par chromatographie sur colonne de silice (éluant : Acétate d'éthyle-Hexane 80/20). On obtient des cristaux blancs. TF = 57-59°C. R = 79 %.
RMN ¹H (CDCl₃, 330°K): 1,81-1,95 (m, 2H, -CH₂-), 2,40-2,48 (t, 2H, -CH₂-), 2,54-2,60 (t, 4H, -H₂C-CH₂-), 2,67-2,72 (t, 2H, -CH₂-), 3,12-3,17 (q, 2H, -CH₂-), 6,06-6,09 (m, 1H, -CH=), 7,24-7,44 (m, 5H, phé). RMN ¹³C (CDCl₃) : 14,7 (-CH₂-CH₂-C N), 22,8 (-CH₂-C N), 27,7 (>C-CH₂-CH₂-N<, pyrid), 50,0 (-CH₂-CH₂-N<, pyrid), 52,9 (=CH-CH₂-N<, pyrid), 55,9 (>N-CH₂-), 119,6 (-CN), 121,3 (>CH-), 124,6 (=CH-, pyri), 126,8 (>(CH)₂-), 128,1 (>CH)₂-), 134,7 (>C=C-, pyri), 140,4 (>C<).

### - N-4-aminobutyl 4-phényl 1,2,3,6 -tétrahydropyridine (C)

Mettre en suspension 1,8 g d'hydrure double de lithium et d'aluminium dans 50 ml de THF anhydre (fraîchement distillé sur sodium) par petites portions (dissolution exothermique). Rajouter 4,5 g de 4-(4-phényl 1,2,3,6-tetrahydropyridinyl) butyronitrile en solution dans du THF au goutte-à-goutte à O°C, puis, laisser réagir à 0°C pendant 3 heures sous bonne agitation. Hydrolyser le milieu avec un mélange de 5 ml d'eau en solution dans 50 ml de THF et laisser reposer une nuit pour agglomérer le précipité. Filtrer le précipité sur célite, sécher le filtrat sur du sulfate de sodium anhydre et concentrer le filtrat. Distiller le résidu sous le vide du moteur. On obtient une huile incolore. R=86 %. RMN ¹H (CDCl₃): 1,26 (s 1, 2H, -NH₂), 1,43-1,66 (m, 4H, -H₂C-CH₂-), 2,44-2,51 (t, 2H, -CH₂-), 2,59 (s 1, 2H, -CH₂-), 2,68-2,76 (m, 4H, -CH₂-CH₂-), 3,15-3,19 (d, 2H, -CH₂-), 3,72-3,78 (q, 2H, -CH₂-), 6,06 (q, 1H, -CH=), 7,22-7,41 (m, 5H, phé). RMN ¹³C (CDCl₃) : 24,6 (-CH₂-CH₂-CH₂-NH₂), 28,1 (-CH₂-CH₂-NH₂), 31,9 (-CH₂-CH₂-N<, pyrid), 42,2 (-CH₂-NH₂), 50,4 (-CH₂-CH₂-N<, pyrid), 53,3 (=CH-CH₂-N<), 58,3 (>N-CH₂-), 121,9 (>CH-), 124,9 (>(CH)₂-), 126,9 (>(CH)₂-), 128,2 (>C=CH-, pyrid), 135,0 (>C=CH-, pyrid), 140,9 (>C<).

### Exemple 3 : Préparation du N-[4-(4-phényl pipéridinyl) butyl] 2-naphtamide (Do 912)

Dans un flacon de PARR de 100 ml, dissoudre 120 mg de N-[4-(4-phényl 1,2,3,6-tetrahydropyridinyl)butyl] 2-naphtamide obtenu selon l'exemple 2, dans 20 ml de méthanol. Rajouter une pointe de spatule de Pd/C (palladium sur charbon) et hydrogénolyser à l'appareil de PARR pendant 6 heures sous une pression de 60 p.s.i.. Filtrer le catalyseur, rincer le catalyseur avec du méthanol et concentrer le filtrat. Purifier par chromatographie sur colonne de silice (éluant : Acétate d'éthyle-Méthanol 90/10). Cristalliser dans de l'hexane. On obtient 110 mg de cristaux blancs. R = 91 %. TF = 143-144°C. RMN ¹H (CDCl₃, 330°K) :
1,76-1,86 (m, 7H, -H₂C-CH₂-CH₂-CH<), 2,06-2,14 (m, 2H, -CH₂-), 2,48-2,54 (m, 4H, -H₂C-CH₂-), 3,07-3,13 (m, 2H, -CH₂-), 3,56-3,60 (q, 2H, -CH₂-), 6,94 (s.l., 1H, -NH), 7,12-7,29 (m, 5H, >(CH)₅-, phényl), 7,51-7,55 (m, 2H, >HC-CH<), 7,84-7,93 (m, 4H, >HC-CH-CH-CH<), 8,30 (s, 1H, >CH-).
Analyse C₂₆H₃₀N₂O
Calc. C % 80,79 ; H % 7,82 ; N % 7,25
Tr. C % 80,66 ; H % 7,89 ; N % 7,22

### Exemple 4 : Préparation du N-[4-(4-(2-méthoxyphényl) pipérazinyl) butyl] 1-méthoxy 4-cyano 2-naphtamide (883)

Dissoudre 120 mg d'acide 1-méthoxy 4-cyano naphtalène 2-carboxylique (préparé comme à l'exemple la)) dans 20 ml d'acétone anhydre. Rajouter 2,5 équivalents de triéthylamine et refroidir le milieu à -15°C au moyen d'un bain carboglace-acétone. Rajouter 1,05 équivalent de chloroformiate d'isobutyle et laisser réagir 1 heure à -15°C. Rajouter ensuite 1,05 équivalent de N-4-aminobutyl N'-(2-méthoxyphényl) pipérazine (préparé comme à l'exemple 1b)) et laisser réagir 2 heures à température ambiante sous atmosphère inerte. Filtrer le chlorhydrate de triéthylamine et évaporer le filtrat à sec. Reprendre le résidu d'évaporation dans un peu d'acétate d'éthyle et purifier le naphtamide par chromatographie sur colonne de silice (éluant : Acétate d'éthyle-Méthanol 90/10). Cristalliser le naphtamide dans du diéthyléther.
On obtient 76 mg de cristaux blancs. R=23%. TF=128°C. RMN ¹³C (CDCl₃) : 24,4 (-CH₂-CH₂-CH₂-NH-), 27,5 (-CH₂-CH₂-NH-), 39,9 (-CH₂-NH-), 50,4 (-CH₂-N-(CH₂)₂-), 53,3 (>C-N-(CH₂)₂-), 55,1 (-OCH₃, phényl), 58,0 (>N-CH₂-), 63,4 (-OCH₃, naphtyl), 106,7 (-CH-C N, ), 111,0 (-CH-C-OCH₃, phényl), 116,9 (-C N), 117,9 (-CH-CH-C-OCH₃, phényl), 120,8 (-CH-C-N<, phényl), 122,5 (>C-C=O,β), 122,8 (-CH-CH-C-N<, phényl), 123,6 (-CH-CH-C-C-OCH₃,β), 125,5 (-CH-C-C-OCH₃,α), 127,5 (-C-C-OCH₃,γ), 128,0 (-CH-C-C N,α), 130,1 (-CH-C-C=O,β), 134,4 (-CH-CH-C-C-C N,β), 134,7 (-C-C N,γ), 141,1 (>C-N<, phényl), 152,1 (-C-OCH₃, phényl), 158,4 (-C-OCH₃,α), 163,8 (>C=O).

### Exemple 5

N-[4-(4-(2-méthoxyphényl) pipérazinyl) butyl] 2-naphtamide TF=121°C, C₂₆H₃₁N₃O₂ (DO 897)

### Exemple 6

N-[4-(4-(2-chlorophényl) pipérazinyl) butyl] 3-méthoxy 2-naphtamide (DO 917)
TF=86°C, C₂₆H₃₀N₃O₂Cl

### Exemple 7

N-[4-(4-(2-chlorophényl) pipérazinyl) butyl] 2-naphtamide TF=107-109°C, C₂₅H₂₈ClN₃O (DO 910)

### Exemple 8

N-[4-(4-(3-chlorophényl) pipérazinyl) butyl] 2-naphtamide TF=150-152°C, C₂₅H₂₈ClN₃O (DO 908)

### Exemple 9

N-[4-(4-phényl pipérazinyl) butyl] 2-naphtamide TF=164°C, C₂₅H₂₉N₃O (DO 905)

### Exemple 10

Oxalate du N-[4-(4-(2-méthoxyphényl) pipérazinyl) butyl] 1-méthoxy 2-naphtamide
TF=164°C, C₂₇H₃₄N₃O₃,C₂H₂O₄ (897a)

### ACTIVITE BIOLOGIQUE

L'activité des dérivés de formule (I) selon l'invention a été évaluée sur des cellules exprimant des récepteurs dopaminergiques humains recombinants dont le degré de stimulation peut être déterminé par la mesure de l'incorporation de thymidine [³H] : cellules CHO exprimant le récepteur D_{2S} et cellules NG 108-15 exprimant le récepteur D₃ (Pilon et al., Eur. J. Pharmacol. Mol. Pharmacol. Sect. 1994, 268: 129-139 ; Sautel et coll. Neuroreport, 1995, 6: 329-332).

Parmi ces dérivés, certains présentent une affinité très supérieure pour le récepteur D₃ par comparaison au récepteur D₂.

Alors que les composés du type nafadotride (demande de brevet français n° 91 13103 précitée) se comportent comme de purs antagonistes du récepteur D₃, les inventeurs ont découvert que, de manière inattendue, les composés de la présente invention se comportent comme de puissants agonistes partiels de la dopamine au récepteur D₃, leur activité intrinsèque variant entre 50 % et 80 % (dopamine = 100 %). C'est ainsi que le composé de l'exemple 5 présente une activité intrinsèque de 60 % et que sa concentration efficace 50 % est de 3 nM. Ce même composé présente une affinité apparente 25 fois plus faible au niveau du récepteur D₂ versus le récepteur D₃ : il constitue dès lors un agoniste (partiel) très sélectif de ce dernier.

Qui plus est, les inventeurs ont également montré que des différences structurales minimes parmi les composés décrits ici, peuvent entraîner des variations importantes dans la sélectivité et l'activité intrinsèque des molécules.

Par exemple, lorsque R₃ et R₄ représentent chacun un groupe méthoxy, cette disubstitution fait perdre la sélectivité récepteur D₃/récepteur D₂ par rapport à une monosubstitution. Par ailleurs, la présence d'un substituant chlore (R₃ ou R₄) diminue considérablement l'affinité du dérivé (I) pour le récepteur D₃.

Ces propriétés conduisent à des applications thérapeutiques non encore envisageables avec les agents dopaminergiques existants. En effet, la haute sélectivité des molécules permet une activation sélective des transmissions dopaminergiques des régions limbiques impliquées dans les processus émotionnels et cognitifs (qui expriment le récepteur D₃) sans interférence avec les transmissions dopaminergiques des systèmes extrapyramidal, antéhypophysaire, ou végétatif (area postrema). Ils doivent donc éviter les effets secondaires des composés existants, liés à l'effet de ces derniers sur les sphères extrapyramidale, antéhypophysaire et végétative. En outre, le caractère agoniste D₃ partiel est de nature à normaliser les transmissions dopaminergiques sans risque d'activation excessive.

Les dérivés de l'invention peuvent donc être utilisés pour la préparation de compositions pharmaceutiques et médicaments pour le traitement d'affections neuropsychiatriques mettant en jeu le récepteur D₃ comme les états psychotiques ou dépressifs.

En outre, compte tenu du rôle du récepteur D₃ dans les états de pharmacodépendance, des compositions pharmaceutiques ou médicaments à base de ces dérivés pourront être utilement administrés dans les états liés à l'abstinence et/ou faciliter la désintoxication de sujets dépendants de la cocaïne, l'héroïne, l'alcool, la nicotine, etc.

Les dérivés selon l'invention possèdent également des effets sur l'érection pénienne et peuvent aussi être utilisés pour la préparation de compositions pharmaceutiques et médicaments pour le traitement de troubles de la sphère sexuelle notamment l'impuissance masculine.

Les dérivés selon l'invention, ainsi que, généralement, les agonistes du récepteur D₃, peuvent également être utilisés pour un traitement complémentaire du traitement de la maladie de Parkinson par la L. DOPA. L'invention concerne donc de tels médicaments complémentaires ainsi que l'utilisation des agonistes du récepteur D₃, y compris les produits nouveaux de la présente invention pour la préparation d'un médicament pour le traitement complémentaire de la maladie de Parkinson.

Cette activité pourrait être expliquée par la découverte, sur un modèle animal de la maladie de Parkinson, que le traitement par la L. DOPA induit l'expression, dans les cellules du striatum, de récepteurs D₃ qui sous-tendraient la sensibilisation aux effets moteurs de la L-DOPA.

Les dérivés de formule (I) selon l'invention peuvent être administrés notamment par voie orale sous forme de composition pharmaceutique.

Les doses thérapeutiquement utiles varient avec les différents dérivés mais, pour le composé de l'exemple 5, on peut préciser qu'elles se situent entre 0,05 et 5 mg/kg par voie orale.

## Revendications

1. Dérivés de 2-naphtamides, sous forme de bases ou de sels, répondant à la formule générale (I) suivante : 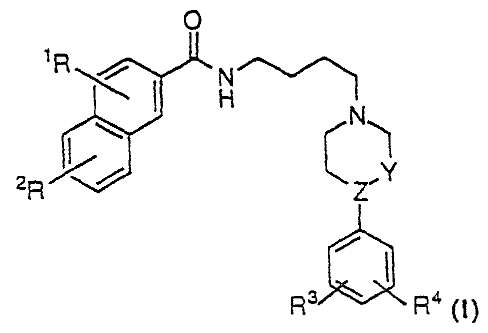 dans laquelle :
- l'entité Z-Y représente un groupement N-CH₂, C=CH ou CH-CH₂ ;
- R¹ représente un atome d'hydrogène, de fluor, de brome ou d'iode, ou un groupement hydroxy, méthoxy, nitrile ou nitro ;
- R² représente un atome d'hydrogène ou de brome, ou un groupement hydroxy, méthoxy, nitrile ou nitro ;
les substituants R₁ et R₂ étant situés tous les deux sur le même noyau du motif naphtamide ou chacun sur un des noyaux ;
- R³ et R⁴ peuvent être identiques ou différents et représentent chacun indépendemment un atome d'hydrogène ou de chlore, ou un groupement méthoxy ou méthyle.

2. Dérivés selon la revendication 1, **caractérisés en ce que** l'entité Z-Y représente un groupement N-CH₂.

3. Dérivés selon la revendication 1, **caractérisés en ce qu'**ils sont choisis parmi les composés suivants :
- N-[4-(4-(2-méthoxyphényl) pipérazinyl) butyl] 1-méthoxy 4-nitro 2-naphtamide ;
- N-[4-(4-phényl 1,2,3,6-tétrahydropyridinyl) butyl] 2-naphtamide ;
- N-[4-(4-phényl pipéridinyl) butyl] 2-naphtamide ;
- N-[4-(4-(2-méthoxyphényl) pipérazinyl) butyl] 1-méthoxy 4-cyano 2-naphtamide ;
- N-[4-(4-(2-méthoxyphényl) pipérazinyl) butyl] 2-naphtamide ;
- N-[4-(4-(2-chlorophényl) pipérazinyl) butyl] 3-méthoxy 2-naphtamide ;
- N-[4-(4-(2-chlorophényl) pipérazinyl) butyl] 2-naphtamide ;
- N-[4-(4-(3-chlorophényl) pipérazinyl) butyl] 2-naphtamide ;
- N-[4-(4-phényl pipérazinyl) butyl] 2-naphtamide ;
- Oxalate du N-[4-(4-(2-méthoxyphényl) pipérazinyl) butyl] 1-méthoxy 2-naphtamide ;

4. Composition pharmaceutique **caractérisée en ce qu'**elle comprend une quantité thérapeutiquement efficace d'au moins un dérivé selon l'une quelconque des revendications 1 à 3, sous forme de base ou de sel pharmaceutiquement acceptable, en combinaison avec un véhicule ou excipient pharmaceutiquement acceptable.

5. Utilisation d'un dérivé selon l'une quelconque des revendications 1 à 3 pour la préparation d'un médicament agissant comme un agoniste partiel du récepteur D₃ de la dopamine.

6. Utilisation selon la revendication 5 pour la préparation d'un médicament pour le traitement d'affections neuropsychiatriques faisant intervenir le récepteur D₃ de la dopamine, notamment pour le traitement des états psychotiques et dépressifs, pour le traitement d'états de pharmacodépendance, notamment les états liés à l'abstinence et/ou à la désintoxication de sujets dépendants de la cocaïne, de l'héroïne, de l'alcool, de la nicotine, etc, ou pour le traitement de troubles de la sphère sexuelle notamment l'impuissance masculine.

7. Utilisation selon la revendication 5 pour la préparation d'un médicament pour un traitement complémentaire de la maladie de Parkinson.

8. Médicament agissant comme agoniste partiel du récepteur D₃ de la dopamine, **caractérisé en ce qu'**il comprend à titre de principe actif, au moins un dérivé selon l'une quelconque des revendications 1 à 3.

9. Médicament selon la revendication 8 pour le traitement d'affections neuropsychiatriques faisant intervenir le récepteur D₃ de la dopamine, notamment pour le traitement des états psychotiques et dépressifs, pour le traitement d'états de pharmacodépendance, notamment les états liés à l'abstinence et/ou la désintoxication de sujets dépendants de la cocaïne, de l'héroïne, de l'alcool, de la nicotine, etc., ou pour le traitement de troubles de la sphère sexuelle, notamment l'impuissance masculine.

10. Médicament selon la revendication 8 pour un traitement complémentaire de la maladie de Parkinson.

11. Utilisation d'un dérivé selon l'une quelconque des revendications 1 à 3 agissant comme un agoniste du récepteur D₃ de la dopamine pour la préparation d'un médicament destiné à un traitement complémentaire de la maladie de Parkinson.

12. Médicament contenant à titre de principe actif, un dérivé selon l'une quelconque des revendications 1 à 3 agissant comme un agoniste du récepteur D₃ de la dopamine, destiné à un traitement complémentaire de la maladie de Parkinson.

## Patentansprüche

1. 2-Naphthamid-Derivate in Form von Basen oder Salzen mit der allgemeinen Formel (I) 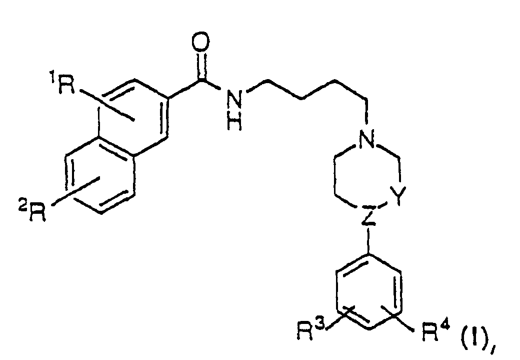 in welcher
- die Einheit Z-Y eine N-CH₂-, C=CH- oder CH-CH₂-Gruppe,
- R¹ ein Wasserstoff-, Fluor-, Brom- bzw. Iodatom oder eine Hydroxy-, Methoxy-, Nitril- bzw. Nitrogruppe und
- R² ein Wasserstoff- bzw. Bromatom oder eine Hydroxy-, Methoxy-, Nitril- bzw. Nitrogruppe bedeutet, wobei
die Substituenten R¹ und R² sich beide an demselben Kern der Naphthamid-Gruppierung oder jeweils an einem der Kerne befinden, und
- R³ und R⁴ gegebenenfalls voneinander verschieden sein können und jeweils unabhängig voneinander ein Wasserstoff- bzw. Chloratom oder eine Methoxy- bzw. Methylgruppe bedeuten.

2. Derivate nach Anspruch 1, **dadurch gekennzeichnet, dass** die Z-Y-Einheit eine N-CH₂-Gruppe bedeutet.

3. Derivate nach Anspruch 1, **dadurch gekennzeichnet, dass** sie aus den Verbindungen
- N-[4-(4-(2-Methoxyphenyl)piperazinyl)butyl]-1-methoxy-4-nitro-2-naphthamid,
- N-[4-(4-Phenyl-1,2,3,6-tetrahydropyridinyl)butyl]-2-naphthamid,
- N- [4-(4-Phenylpiperidinyl)butyl]-2-naphthamid,
- N-[4-(4-(2-Methoxyphenyl)piperazinyl)butyl]-1-methoxy-4-cyano-2-naphthamid,
- N-[4-(4-(2-Methoxyphenyl)piperazinyl)butyl]-2-naphthamid,
- N-[4-(4-(2-Chlorphenyl)piperazinyl)butyl]-3-methoxy-2-naphthamid,
- N-[4-(4-(2-Chlorphenyl)piperazinyl)butyl]-2-naphthamid,
- N- [4-(4- (3-Chlorphenyl)piperazinyl)butyl]-2-naphthamid,
- N-[4-(4-Phenylpiperazinyl)butyl]-2-naphthamid und
- N- [4- (4-(2-Methoxyphenyl)piperazinyl)butyl] -1-methoxy-2-naphthamidoxalat
ausgewählt sind.

4. Pharmazeutische Zusammensetzung, **dadurch gekennzeichnet, dass** sie eine therapeutisch wirksame Menge mindestens eines Derivats nach einem der Ansprüche 1 bis 3 in Form einer pharmazeutisch verträglichen Base oder eines pharmazeutisch verträglichen Salzes in Kombination mit einer pharmazeutisch verträglichen Grundlage oder einem pharmazeutisch verträglichen Träger umfasst.

5. Verwendung eines Derivats nach einem der Ansprüche 1 bis 3 zur Herstellung eines Arzneimittels, das am D₃-Dopamin-Rezeptor als partieller Agonist wirkt.

6. Verwendung nach Anspruch 5 zur Herstellung eines Arzneimittels für die Behandlung neuropsychiatrischer Erkrankungen, an denen der D₃-Dopamin-Rezeptor beteiligt ist, insbesondere für die Behandlung psychotischer und depressiver Zustände, für die Behandlung von medikamentenabhängigen Zuständen, speziell Zuständen, die mit dem Entzug und/oder der Entgiftung von Personen verbunden sind, die von beispielsweise Kokain, Heroin, Alkohol und Nikotin abhängig sind, oder für die Behandlung sexueller Funktionsstörungen, insbesondere der männlichen Impotenz.

7. Verwendung nach Anspruch 5 zur Herstellung eines Arzneimittels für eine ergänzende Behandlung des Parkinson-Syndroms.

8. Arzneimittel, das am D₃-Dopamin-Rezeptor als partieller Agonist wirkt, **dadurch gekennzeichnet, dass** es als Wirkstoff mindestens ein Derivat nach einem der Ansprüche 1 bis 3 enthält.

9. Arzneimittel nach Anspruch 8 für die Behandlung neuropsychiatrischer Erkrankungen, an denen der D₃-Dopamin-Rezeptor beteiligt ist, insbesondere für die Behandlung psychotischer und depressiver Zustände, für die Behandlung von medikamentenabhängigen Zuständen, speziell Zuständen, die mit dem Entzug und/oder der Entgiftung von Personen verbunden sind, die von beispielsweise Kokain, Heroin, Alkohol und Nikotin abhängig sind, oder für die Behandlung sexueller Funktionsstörungen, insbesondere der männlichen Impotenz.

10. Arzneimittel nach Anspruch 8 für eine ergänzende Behandlung des Parkinson-Syndroms.

11. Verwendung eines Derivats nach einem der Ansprüche 1 bis 3, das am D₃-Dopamin-Rezeptor als Agonist wirkt, zur Herstellung eines Arzneimittels, das für eine ergänzende Behandlung des Parkinson-Syndroms vorgesehen ist.

12. Arzneimittel, das als Wirkstoff ein am D₃-Dopamin-Rezeptor als Agonist wirkendes Derivat nach einem der Ansprüche 1 bis 3 enthält und für eine ergänzende Behandlung des Parkinson-Syndroms vorgesehen ist.

## Claims

1. Derivatives of 2-naphthamides, in the form of bases or of salts, fulfilling the following general formula (I): 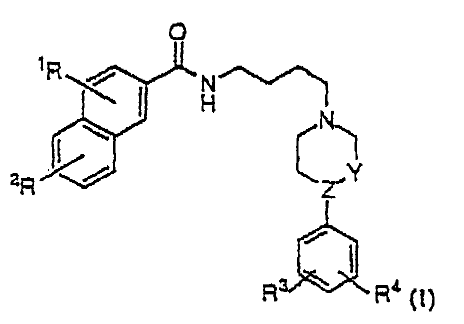 in which:
- the entity Z-Y represents a N-CH₂, C=CH or CH-CH₂ group;
- R¹ represents an atom of hydrogen, fluorine, bromine or iodine, or a hydroxy, methoxy, nitrile or nitro group;
- R² represents an atom of hydrogen or bromine, or a hydroxy, methoxy, nitrile or nitro group;
the substituants R₁ and R₂ being both situated on the same nucleus of the naphthamide unit or each on one of the nuclei;
- R³ and R⁴ can be identical or different and each represent independently an atom of hydrogen or chlorine, or a methoxy or methyl group.

2. Derivatives according to claim 1, **characterised in that** the Z-Y entity represents a N-CH₂ group.

3. Derivatives according to claim 1, **characterised in that** they are chosen from among the following compounds:
- N-[4-(4-(2-methoxyphenyl)piperazinyl)butyl]1-methoxy 4-nitro 2-naphthamide;
- N-[4-(4-phenyl 1,2,3,6-tetrahydropyridinyl)butyl]2-naphthamide;
- N-[4-(4-phenylpiperidinyl)butyl]2-naphthamide;
- N-[4-(4-(2-methoxyphenyl)piperazinyl)butyl]1-methoxy 4-cyano 2-naphthamide;
- N-[4-(4-(2-methoxyphenyl)piperazinyl)butyl]2-naphthamide;
- N-[4-(4-(2-chlorophenyl)piperazinyl)butyl]3-methoxy 2-naphthamide;
- N-[4-(4-(2-chlorophenyl)piperazinyl)butyl]2-naphthamide;
- N-[4-(4-(3-chlorophenyl)piperazinyl)butyl]2-naphthamide;
- N-[4-(4-phenylpiperazinyl)butyl]2-naphthamide;
- N-[4-(4-(2-methoxyphenyl)piperazinyl)butyl]1-methoxy 2-naphthamide oxalate;

4. Pharmaceutical composition **characterised in that** it comprises a therapeutically efficacious quantity of at least one derivative according to any of the claims 1 to 3, in the form of a pharmaceutically acceptable base or salt, in combination with a pharmaceutically acceptable vehicle or excipient.

5. Use of a derivative according to any of claims 1 to 3 for preparation of a medication acting as a partial agonist of the D₃ dopamine receptor.

6. Use according to claim 5 for preparation of a medication for the treatment of neuropsychiatric disorders, by the intervention of the D₃ dopamine receptor, in particular for the treatment of psychotic and depressive conditions, for the treatment of conditions of drug dependency, in particular conditions related to abstinence and/or to detoxification of patients who are dependent upon cocaine, heroin, alcohol, nicotine etc. or for the treatment of problems in the sexual sphere, particularly male impotence.

7. Use according to claim 5 for the preparation of a medication for a complementary treatment of Parkinson's disease.

8. Medication acting as a partial agonist of the D₃ dopamine receptor, **characterised in that** it comprises, by way of active principle, at least one derivative according to any of the claims 1 to 3.

9. Medication according to claim 8 for the treatment of neuropsychiatric disorders by the intervention of the D₃ dopamine receptor, in particular for the treatment of psychotic and depressive conditions, for the treatment of conditions of drug dependency, in particular conditions related to abstinence and/or detoxification of patients who are dependent upon cocaine, heroin, alcohol, nicotine etc. or for the treatment of disorders in the sexual sphere, particularly male impotence.

10. Medication according to claim 8 for a complementary treatment of Parkinson's disease.

11. Use of a derivative according to any of claims 1 to 3 acting as an agonist of the D₃ dopamine receptor, for the preparation of a medication intended for a complementary treatment of Parkinson's disease.

12. Medication which contains, by way of active principle, a derivative according to any of claims 1 to 3 as an agonist of the D₃ dopamine receptor, intended for a complementary treatment of Parkinson's disease.
